# EUROPEAN PATENT SPECIFICATION

(11) **EP 2 700 718 B1**
(45) Date of publication and mention of the grant of the patent: **18.05.2016**
(21) Application number: 12757192.5
(22) Date of filing: 12.03.2012
(51) Int. Cl.: C12Q 1/56, G01N 33/68

(54) **METHOD FOR DETECTING PROTEIN S DEFICIENCY**
VERFAHREN ZUR ERKENNUNG VON PROTEIN-S-MANGEL
PROCÉDÉ DE DÉTECTION D'UNE DÉFICIENCE EN PROTÉINE S

(30) Priority: 14.03.2011 JP 2011056019
(43) Date of publication of application: 26.02.2014
(73) Proprietor: School Corporation Kyushu Bunka Gakuen, Sasebo-shi Nagasaki 859-3243 (JP)
(72) Inventor: HAMASAKI, Naotaka, Sasebo-shi, Nagasaki 859-3298 (JP); TSUDA, Tomohide, Sagamihara-shi, Kanagawa 252-0331 (JP); JIN, Xiuri, Sagamihara-shi, Kanagawa 252-0331 (JP)
(74) Representative: Schäfer, Matthias W.
(86) International application number: PCT/JP2012/056288
(87) International publication number: WO 2012/124654

(56) References cited:
- WO-A1-2010/128146
- JP-A- 2004 337 143
- H P SCHWARZ ET AL: "Low total protein S antigen but high protein S activity due to decreased C4b-binding protein in neonates", BLOOD, vol. 71, 1 March 1988 (1988-03-01), pages 562-565, XP055148938, ISSN: 0006-4971
- MAKOTO IKEJIRI ET AL.: 'Kessensho Shiketsu Ijosho Shinryo Center ni Okeru Idenshi Kaiseki' THERAP. RES. vol. 29, no. 5, 2008, pages 668 - 670, XP008170885
- KYOTARO IDE ET AL.: 'Haikosoku o Kurikaeshita Protein S Bunshi Ijosho no Ichirei' THE JOURNAL OF THE JAPANESE RESPIRATORY SOCIETY vol. 37, no. 5, 1999, pages 410 - 414, XP008170890
- SHUNICHI KIN ET AL.: 'A new fully automated assay to quantify total protein S' CLINICAL TESTING vol. 55, no. 4, 15 April 2011, pages 393 - 398, XP008170886
- TSUDA T. ET AL.: 'New quantitative total protein S-assay system for diagnosing protein S type II deficiency: clinical application of the screening system for protein S type II deficiency' BLOOD COAGUL. FIBRINOLYSIS vol. 23, no. 1, January 2012, pages 56 - 63, XP055125429

## Description

### [Field of the Invention]

The present invention relates to a method of detecting protein S abnormalities.

The invention is especially useful in the fields of laboratory tests, molecular biology, and medicine.

### [Background of the Invention]

Protein S is a plasma protein that primarily works in a control mechanism of an in vivo blood coagulation system.

Protein S is primarily present in blood, is a prosthetic factor (cofactor) of activated protein C, is capable of increasing activity of activated protein C, and is indispensable for action of activated protein C in blood.

Here, "activated protein C" is a factor that plays a role of suppressing a blood coagulation reaction by decomposition of activated blood coagulation factor V (Factor Va; FVa), and activated blood coagulation factor VIII (Factor VIIIa; FVIIIa), which promote blood coagulation in human.

Protein S specifically makes a one-to-one binding with C4b-binding protein (a complement factor 4b-binding protein; C4bBP) to form a complex. In other words, C4b-binding protein becomes a ligand for protein S.

The complex-forming reaction between protein S and C4b-binding protein is as follows, and is a reversible reaction.

[Chemical Formula 1] **Protein S + C4b-binding protein ← → Protein S-C4b-binding protein complex**

In human blood, the molarity of C4b-binding protein is typically less than that of protein S and the dissociation constant is also small, so that in blood, there are only present "protein S " and "protein S-C4b-binding protein complex. In other words, the equilibrium of the above reaction is completely dominant on the right side.

Normally, among protein S in blood (in plasma) of healthy individual, about 60% thereof is "protein S -C4b-binding protein complex" (i.e., bound form), and about 40% thereof is "protein S in the free state" (i.e., free form).

Here, only free protein S (i.e., free form) exhibits coenzyme activity to activated protein C and is capable of increasing activity of activated protein C.

Here, in the specification, unless there is description of whether it is a complex (or bound form), free (free form), or the like, the terms, "protein S" or "C4b-binding protein", or the like collectively mean their complexes (or bound form) and the ones in the free state (or free form) of the respective substances.

A schematic diagram of a regulatory mechanism of a blood coagulation system by protein C and protein S is show below.

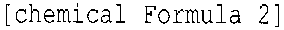

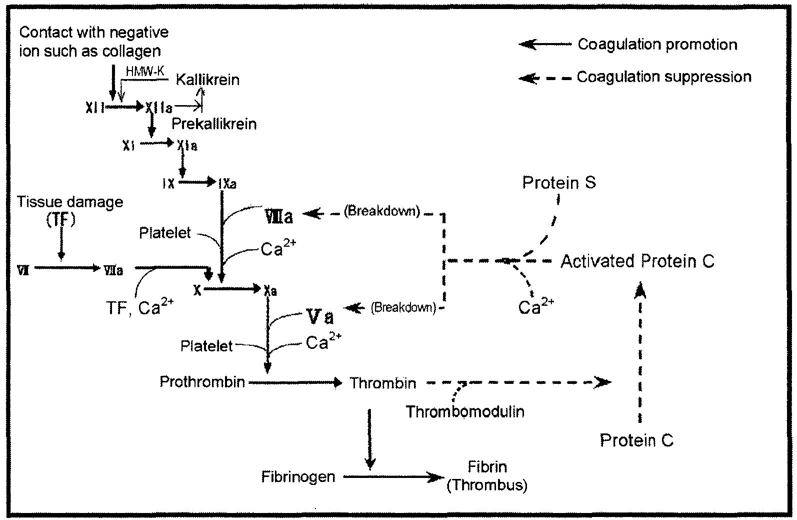

Inside the living body, protein C is subjected to limited breakdown by thrombin-thrombomodulin complex, releases activated peptide, and thereby becomes activated, and turns into activated protein C.

Activated protein C is a serine protease, which serves for suppressing the blood coagulation reaction by breaking down the activated blood coagulation factor V that promotes blood coagulation in humans and the activated blood coagulation factor VIII.

Protein S is a prosthetic factor (cofactor) of activated protein C, and the activity of activated protein C increases with presence of protein S, and the breakdown reaction of activated blood coagulation factor V by the activated protein C and the breakdown reaction of the activated blood coagulation factor VIII are promoted.

Decrease or abnormality of activity of protein S, which has a function of suppressing blood coagulation reaction, could become a cause of thrombosis in the living bodies.

Actually, patients with congenital protein S abnormalities highly frequently develop venous thrombosis such as deep venous thrombosis, superficial phlebitis, and pulmonary infarction, or arterial thrombosis such as coronary thrombosis that could cause heart failure, and the like.

In addition, also in case of disseminated intravascular coagulation (DIC), vitamin K deficiency, hypohepatia, or the like, decrease or abnormality of protein S activity is recognized.

More specifically, by measuring activity of protein S in a specimen such as plasma, it is possible to find out decrease or abnormality of protein S activity, which in turn can serve for prediction of onset, early detection, and determination of treatment results of diseases such as thrombosis, or the like.

As a method of measuring activity of protein S in a specimen, there is disclosed a method that includes the steps of: incubating a specimen with a protein C-activator from snake poison under formation of activated protein C; adding an activator of a blood coagulation factor XII, a blood coagulation factor VII, or a blood coagulation factor II (prothrombin); and photometrically measuring the decrease of formation of thrombin from prothrombin, which is mediated by a blood coagulation factor and the activator, using a chromogenic thrombin substrate (see Patent Reference 1).

As another method of measuring activity of protein S in a specimen, there is disclosed a method, which includes the steps of: adding in a plasma specimen a protein C-activating substance that activates protein C or activated protein C; then adding activated blood coagulation factor IX and incubating; measuring the amount of produced thrombin by a known method; and comparing with the value obtained for a standard specimen with known protein S activity and determining the activity of protein S in the specimen (see Patent Reference 2).

Furthermore, as another method of measuring activity of protein S in a specimen, there is disclosed a method of measuring activity of protein S in a specimen, which includes the steps of incubating a specimen with activated protein C, a blood coagulation factor VIII, phospholipid and calcium ions; subsequently incubating the mixture with activated blood coagulation factor II (thrombin), activated blood coagulation factor IX, and activated blood coagulation factor X; then adding an activated blood coagulation factor X-specific substrate into the incubated mixture; and measuring the amount of signals produced by cleavage of the substrate (see Patent Reference 3).

In those conventional activity assay methods and activity assay reagents for protein S in a specimen, at least one of components (factors) in blood coagulation reaction to use in the assay reaction is a component (factor) contained in a specimen and used as is. In other words, they depend on components contained in the specimen.

Therefore, in case of conventional activity assay methods and activity assay reagents for protein S in a specimen, in which a component (factor) contained in a specimen is used as is, when the component (factor) [e.g., activated blood coagulation factor X] is deficient or decreased, at least one of the reactive components (factors) of the assay reaction is not present in a sufficient amount, so that the assay reaction does not fully progress and there is an issue that the protein S activity value obtained may be off from the original value and may contain errors in some cases.

In other words, there is a problem that the measured value is affected by the amount (concentration), which is present in a specimen, or variation of a component (factor) that involves in the blood coagulation reaction.

Therefore, the present inventors invented and disclosed an activity assay reagent for protein S contained in a specimen, which contains activated protein C, phospholipid, calcium ions, activated blood coagulation factor V, activated blood coagulation factor X, prothrombin and a substrate of thrombin, which are not derived from the specimen, and also an activity assay method for protein S in a specimen, whereby an activity value of protein S contained in the specimen is obtained by: contacting a specimen with activated protein C, phospholipid, calcium ions, activated blood coagulation factor V, activated blood coagulation factor X, prothrombin and a substrate of thrombin, which are not derived from the specimen; subsequently measuring the amount of signals produced from the substrate of thrombin as a result of reactions of the respective components described above; and then determining the amount of signals suppressed from production according to the activity of protein S contained in the specimen (see Patent Reference 4 and Non-Patent Reference 1).

However, any of the conventional activity assay methods and activity assay reagents for protein S in a specimen is for measuring activity of free protein S (free form).

More specifically, with any of the conventional activity assay methods and activity assay reagents for protein S in a specimen, it is impossible to measure any protein S present in a specimen [a complex of protein S and C4b-binding protein (bound form) and free protein S (free form)], i.e. total protein S activity.

It has been known that Protein S abnormalities repeatedly develop thrombosis, and especially there are many reports that protein S gene mutations are highly frequently observed in Asians.

For example, one of them is protein S Tokushima (PS-K155E gene mutation), which has attracted attentions as one of risk factors of venous thromboembolism (VTE) for Japanese.

Here, molecular abnormality of protein S caused by gene mutation such as protein S Tokushima (PS-K155E gene mutation) is categorized as protein S Type II disorder, in which protein S activity decreases while the protein content of protein S is normal.

Detection of protein S abnormalities accompanied by decrease of protein S activity is considered to serve for prevention, diagnosis, and treatment of thrombosis such as venous thromboembolism.

However, it has been reported that it is difficult to conveniently and accurately detect the protein S abnormalities with conventional methods and reagents (see Non-Patent Reference 2).

### [Prior Art][Patent References]

[Patent Reference 1] Japanese Patent Application Publication S62-212569
[Patent Reference 2] Japanese National Patent Application of Translated Version H4-506603
[Patent Reference 3] Japanese National Patent Application of Translated Version H6-504682
[Patent Reference 4] Japanese Patent Application Publication 2004-337143
[Non-Patent References][0031][Non-Patent Reference 1] Clin. Chem. Lab. Med., 2004, Vol. 42, No. 3, p. 350-352
[Non-Patent Reference 2] Journal of Thrombosis and Homeostasis, 2006, Vol. 4, p. 2010-2013

### [Summary of the Invention]

### [Problems to be solved by the Invention]

An object of the invention is to provide a method of accurately, conveniently, and quickly detecting protein S abnormalities.

### [Means to Solve the Problems]

Deliberately conducting studies on a method of detecting protein S abnormalities, the present inventors found that it is possible to solve the problems by measuring a total Protein activity value and total protein content of protein S in a specimen and comparing them, and completed the invention.

More specifically, the invention includes the following aspects:
(1) A method of detecting protein S abnormalities, which includes the steps of: measuring a total protein S activity value and a protein content of total protein S in a specimen; and comparing the total protein S activity value obtained in the measurement with the protein content of total protein S.
(2) The method of detecting protein S abnormalities according to the aforementioned (1), which further comprising the step of, when the total protein S activity value obtained by the measurement and the protein content of total protein S are compared, and there is a gap between the total protein S value and the protein content of total protein S, judging as the specimen has or is suspected to have the protein S abnormalities; and
(3) The method of detecting protein S abnormalities according to the aforementioned (1) or (2), in which the comparison between the total protein S activity value obtained by the measurement and the protein content of total protein S is determination of a specific activity that is obtained by dividing the total protein S activity value with the protein content of total protein S.

### [Effects of the Invention]

According to the method of detecting protein S abnormalities of the invention, it is possible to accurately, conveniently, and quickly detect protein S abnormalities.

Therefore, with the method of detecting protein S abnormalities of the invention, it is possible to conveniently and accurately detect protein S abnormalities, and to use for prevention, diagnosis, treatment, or the like of diseases such as thrombosis.

### [Brief Description of the Drawings]

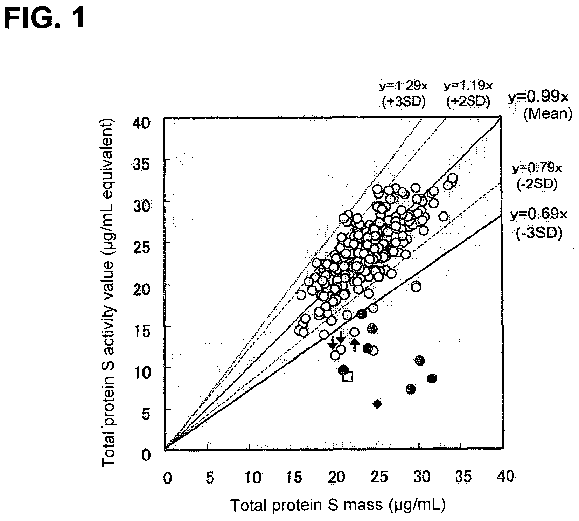

Fig. 1 shows a comparison between the measurement result of the activity value of total protein S in a specimen and the measurement result of protein content of total protein S in the specimen.

### [Embodiments of the Invention]

### I. Overview of Protein S Abnormality Detection Method

The method of detecting protein S abnormalities of the invention includes the steps of: measuring an activity value of total protein S and protein content of total protein S in a specimen; and comparing between the activity value of total protein S and the protein content of total protein S, which are obtained by the measurement.

Here, "measurement of an activity value of total protein S" means measurement of any protein S present in the specimen [a complex of protein S and C4b-binding protein (bound type) and free protein S (free form)].

In other words, in addition to activity of "free protein S (free form)", the activity to measure includes the one that would be observed when protein S contained in the "complex of protein S and C4b-binding protein (bound type)" becomes free protein S (free form).

Moreover, the "measurement of protein content of total protein S means measurement of protein content of any protein S present in the specimen [a complex of protein S and C4b-binding protein (bound type) and free protein S (free form)].

In other words, in addition to protein content of "free protein S (free form)", the protein content of protein S to measure includes the one contained in the "complex of protein S and C4b-binding protein (bound type)".

Here, according to the invention, a method (activity assay method) and reagent (activity assay reagent) for measuring a total protein S activity value are not specifically limited, and can be any as long as they are a method and a reagent whereby it is possible to measure an activity value of total protein S.

Furthermore, according to the invention, the method (assay method) and reagent (assay reagent) for measuring protein content of total protein S are not specifically limited, and can be any as long as they are a method and a reagent whereby it is possible to measure the protein content of total protein S.

The next is the step of comparing between the activity value of total protein S and the protein content of total protein S in the specimen, and a method of such comparison can be suitably decided and is not specifically limited.

A method for the comparison may include, for example, comparing between a numerical value of total protein S activity obtained by the measurement and a numerical value of protein content of total protein S obtained by the measurement; comparing by plotting the total protein S activity obtained by the measurement as a vertical coordinate (can be horizontal coordinate) of the graph, plotting the protein content of the total protein S obtained by the measurement as a horizontal coordinate (can be vertical coordinate) of the graph, and comparing them on the graph; and determining specific activity, which is obtained by dividing the total protein S activity obtained from the measurement with the total protein S protein content obtained from the measurement.

According to the invention, the total protein S activity and the total protein S protein content, which are obtained from the measurement are preferably compared by determining specific activity obtained by dividing the total protein S activity with the total protein S protein content.

Here, according to the invention, for example, when the total protein S activity and the total protein S protein content, which are obtained from the measurements, are compared and there is a gap between the total protein S activity and the total protein S protein content, it is possible to assume that the specimen has or is suspected to have protein S abnormalities.

For example, when a numerical value of the total protein S activity obtained from measurement and a numerical value of the total protein S protein content obtained from measurement are compared and there is a gap therebetween, it is possible to assume that the specimen has or is suspected to have protein S abnormalities.

In addition, for example, when the total protein S activity obtained from measurement is plotted as vertical coordinate (or can be horizontal coordinate) and total protein S protein content obtained from measurement is plotted as horizontal coordinate (or can be vertical coordinate), and there is a gap between the total protein S activity and the total protein S protein content as a result of judgment on the graph, it is considered to have or to be suspected to have protein S abnormalities.

For example, when a point (data) plotted as described above is not on the line (equation) of y = x and is deviated from the line (equation) of y = x, it is also possible to judge as there is the gap.

Furthermore, alternatively, measuring the total protein S activity and the total protein S protein content for individuals who are already known not to have protein S abnormalities, plotting the total protein S activity obtained from the measurement and the total protein S protein content on the graph as described above, comparing them with a group (or an equation expressing the group) of points (data) plotted for specimens from individuals who are known not to have protein S abnormalities, when there is deviation from the group (or an equation expressing the group), it is possible to judge as there is a gap.

In addition, for example, determining specific activity that is obtained by dividing the total protein S protein activity obtained from the measurement with the total protein S protein content obtained from the measurement so as to compare the total protein S protein activity obtained from the measurement with the total protein S protein content, when there is a gap between the total protein S activity and the total protein S protein content, it is possible to consider that the individual has or is suspected to have protein S abnormalities.

Here, determination of specific activity by dividing the total protein S protein activity obtained from the measurement with the total protein S protein content obtained from the measurement can be also done, for example, by dividing the numerical value of the total protein S protein activity obtained from the measurement with the numerical value of the total protein S protein content obtained from the measurement; but alternatively, the specific activity can be also determined by plotting the total protein S protein activity obtained from the measurement as a vertical coordinate in a graph and plotting the total protein S protein content obtained from the measurement as a horizontal coordinate in the graph so as to prepare a graph, or the like.

Moreover, it is also possible to judge the presence of a gap when the specific activity is determined by dividing the total protein S protein activity obtained from the measurement with the total protein S protein content obtained from the measurement as described above, and the value of the specific activity is not 1, but the value slightly off from 1.

In addition, it is also possible to judge the presence of a gap by measuring the total protein S activity and total protein S protein content for individuals who are known not to have protein S abnormalities; determining specific activity by dividing the total protein S activity obtained from the measurement with the total protein S protein content obtained from the measurement; and comparing with the specific activity of a group (or an equation expressing the group) of individuals who are known not to have protein S abnormalities to see whether there is deviation from the group (or an equation expressing the group).

Here, as for judging the presence of a gap when comparison is made with the group (or the equation expressing the group) of specific activity of specimens from individuals who are known not to have protein S abnormalities and there is deviation from the group (or the equation expressing the group), it may be possible to judge as there is a gap when there is deviation from the range that is twice the standard deviation (more preferably, the range three times the standard deviation) of the specific activity of specimens from individuals who are known not to have protein S abnormalities, since the data are off from the group.

In addition, for example, by plotting the total protein S protein activity obtained from the measurement as a vertical coordinate in a graph and plotting the total protein S protein content obtained from the measurement as a horizontal coordinate in the graph or by other methods to show the specific activity on a graph, when it is possible to judge as there is a gap between the total protein S activity and the total protein S protein content from the specific activity shown in the graph, it is possible to consider that the individual has or is suspected to have protein S abnormalities.

For example, when a point (data) of specific activity plotted on the graph as described above is not on the line (equation) of y = x and is off from and under the line (equation) of y = x, it is considered as there is a gap, and it is also possible to consider that the individual has or is suspected to have protein S abnormalities.

In addition, alternatively, when the total protein S activity and the total protein S protein content are measured for individuals who are known not to have protein S abnormalities, the total protein S protein activity obtained from the measurement and the total protein S protein content are plotted on a graph as described above so as to show the specific activity on the graph, comparison is made with the group (or an equation expressing the group) of points (data) of specimens from individuals who are known not to have protein S abnormalities, and there is deviation from the group (or equation expressing the group) being therebelow, it is judged as there is a gap, and it is possible to consider as the individual has or is suspected to have protein S abnormalities.

Here, as for judging the presence of a gap when the total protein S activity and the total protein S protein content are measured for individuals who are known not to have protein S abnormalities; the total protein S protein activity obtained from the measurement and the total protein S protein content are plotted on the graph as described above so as to show the specific activity on the graph; comparison is made with the group (or equation expressing the group) of points
(data) of specific activity of specimens from individuals who are known not to have protein S abnormalities; and there is deviation from the group (or equation expressing the group), it is also possible to judge as there is a gap when the mean value and standard deviation (hereinafter, may be referred to as "SD") are determined from the group of points (data) of specific activity plotted for specimens from individuals who are known not to have protein S abnormalities, and when the specific activity of a specimen is not greater than the mean - 2SD (more preferably, mean - 3SD), it can be considered as there is deviation and a gap from the group.

### II. Method of Measuring Total protein S Activity in a Specimen

As described above, according to the invention, a method (activity assay method) and a reagent (activity assay reagent) for measuring total protein S activity are not specifically limited, and can be any as long as they are a method and a reagent whereby it is possible to measure an activity value of total protein S, but hereunder, examples of activity assay methods for total protein S in a specimen will be described.

### I. General

In an activity assay method for total protein S in a specimen, it is preferred to have a surfactant present upon reaction for measuring the total protein S activity.

Here, in the activity assay method for total protein S in a specimen, it is preferred to have a surfactant present upon reaction, in which activity of activated protein C increases by protein S, and upon reaction, in which activated blood coagulation factor V is broken down by the activity-enhanced activated Protein C.

In addition, in the activity assay method for total protein S in a specimen, it is preferred to have phospholipid that is composed of phosphatidylcholine, phosphatidylserine and phosphatidylethanolamine upon activity assay reaction for total protein S.

Then, in the activity assay method for total protein S in a specimen, upon reaction, in which the activity of activated protein C increases by protein S, and upon reaction, in which the activated blood coagulation factor V is broken down by the activity-enhanced activated protein C, it is preferred to have phospholipid present, which is composed of phosphatidylcholine, phosphatidylserine and phosphatidylethanolamine.

The activity assay method for total protein S in a specimen includes the following steps (a) to (c):
(a) Contact a specimen with activated protein C, the surfactant, the phospholipid, calcium ions, activated blood coagulation factor V, activated blood coagulation factor X, prothrombin and a substrate of thrombin;
(b) Subsequently, measure the amount of signals produced from the substrate of thrombin as a result of reactions of the respective components in the above-described (a); and
(c) Then, with the amount of signals, production of which is suppressed according to the activity of total protein S contained in the specimen, obtain the activity of total protein S contained in the specimen.

In addition, in the activity assay method for total protein S in a specimen, the measurement of an activity value of total protein S in a specimen is preferably performed by a method that includes the following steps (a) to (e).
(a) By contacting a specimen with at least activated protein C, surfactant, and phospholipid and calcium ions, and if the specimen contains protein S, the activity of activated protein C increases;
(b) Thereafter, in the presence of the components in the above-described (a) and activated blood coagulation factor V, the breakdown reaction of activated blood coagulation factor V, which is catalyzed by activated protein C, is promoted by the increase of activity of the activated protein C in the above-described (a);
(c) Subsequently, the reaction to produce thrombin from prothrombin that is catalyzed by the activated blood coagulation factor X in the presence of phospholipid and calcium ions, with the reaction is promoted by activated blood coagulation factor V, is suppressed by promotion of the breakdown reaction of the activated blood coagulation factor V in the above-described (b), and the production of thrombin is suppressed;
(d) Then, with decrease in production of thrombin in the above-described (c), the reaction that produces signals from the substrate of thrombin, which is catalyzed by thrombin, is suppressed.
(e) Thereafter, measuring the amount of signals that are suppressed from production by suppression of the reaction in the above-described (d), the activity of the total protein S contained in the specimen is obtained.

### 2. Method of Measuring Activity of the Total protein S in a Specimen

An assay method for measuring activity of total protein S in a specimen includes the methods described in the following (1) or (2), and it is preferred to perform the measurement by those methods.
(1)
   (a) Contact a specimen with activated protein C, a surfactant, phospholipid, calcium ions, activated blood coagulation factor V (Factor Va; FVa), activated blood coagulation factor X (Factor Xa; FXa), prothrombin and a substrate of thrombin.
      Here, as for the contact of a specimen with those components, it is possible to contact with all the components at once so as to perform all the reactions in the following reaction system in one step (1-step method), or further, it is also possible to divide the above-described components and perform the contact dividing into a plurality of steps so as to perform the reactions of the following reaction system dividing them into a plurality of steps (multi-step method).
   (b) By the contact in the above-described (a), the following reactions by each of the above-described component progress.
      Then, the amount of signals produced from the substrate of thrombin through the series of reactions is measured.
      More specifically, the amount of signals (e.g. absorbance) is measured, which are produced as a result of that the substrate of thrombin is subjected to actions such as breakdown by thrombin by the series of the above-described reactions.
   (c) When the specimen contains protein S, the activity of activated protein C becomes enhanced in the presence of phospholipid and calcium ions, and thereby the breakdown of activated blood coagulation factor V is promoted, so that the reaction that produces signals from the substrate of thrombin, which is catalyzed by thrombin, is suppressed and production of signals is suppressed.

   The degree of suppression of the signal production increases according to the activity of total protein S contained in the specimen.
   Therefore, measuring the amount of signals in the above-described (b), the amount of signals suppressed from production is measured, and thereby it is possible to obtain the activity of total protein S contained in the specimen. 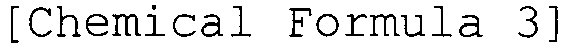 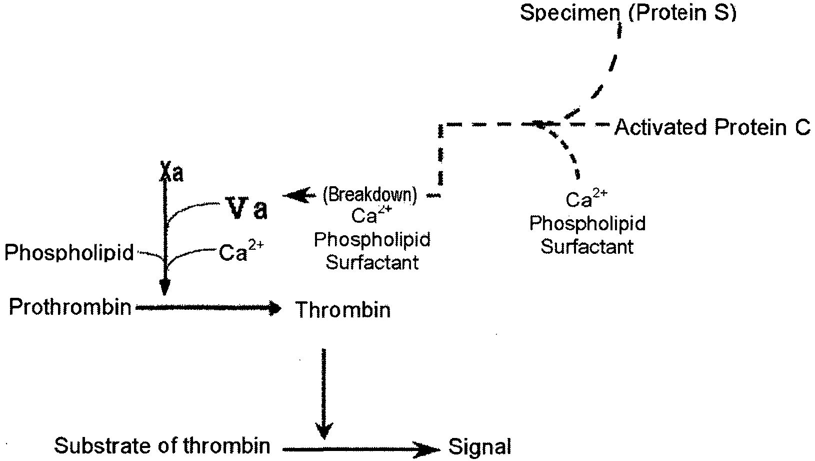
(2)
   The method is for measuring activity of protein S in a specimen using the following reaction system composed of activated protein C, surfactant, phospholipid, calcium ions, activated blood coagulation factor V (Factor Va; FVa), activated blood coagulation factor X (Factor Xa; FXa), prothrombin and a substrate of thrombin;
   (a) The specimen is contacted with at least activated protein C, surfactant, phospholipid and calcium ions.
      With this procedure, when the specimen contains protein S, the activity of the activated protein C increases in the presence of phospholipid and calcium ions.
   (b) With increase of activity of activated protein C in the above-described (a), the breakdown reaction of activated blood coagulation factor V that is catalyzed by activated protein C in the presence of phospholipid and calcium ions is promoted in the co-presence of a surfactant.
   (c) The reaction to produce thrombin from prothrombin that is catalyzed by activated blood coagulation factor X in the presence of phospholipid and calcium ions, which is promoted by activated blood coagulation factor V, is suppressed by promotion of the breakdown reaction of activated blood coagulation factor V in the above-described (b), and production of thrombin decreases.
   (d) With decrease of production of thrombin in the above-described (c), the reaction that produces signals from the substrate of thrombin, which is catalyzed by thrombin, is suppressed.
      Here, the degree of suppression of the signal production increases according to the activity of total protein S contained in the specimen.
   (e) The amount of signals that are suppressed from production by suppression of the reaction in the above-described (d) is measured.

As described above, measuring the amount of signals produced from the above-described reaction, i.e. measuring the amount of signals that is suppressed from production, it is possible to obtain the activity of total protein S contained in the specimen.

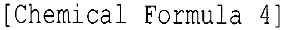

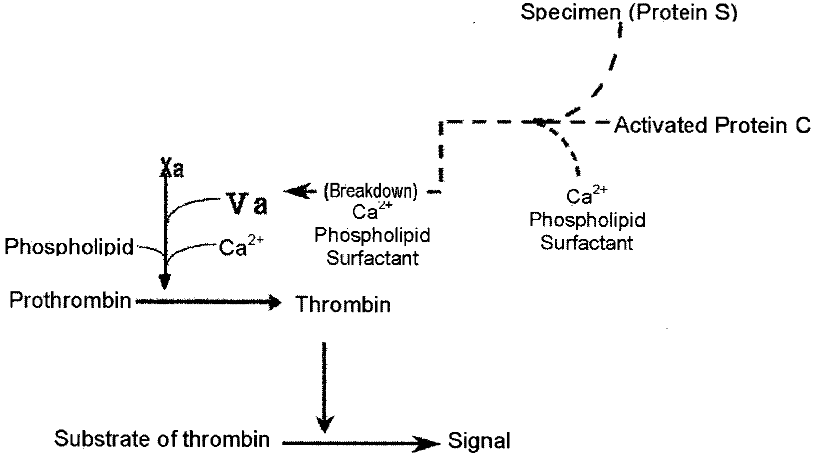

### 3. Specimens

According to the activity assay method for total protein S in a specimen, the specimen means a substance that may contain protein S.

Such specimen may include, for example, biological specimens (specimens derived from human or animals).

Such biological specimens may include, for example, biological fluids such as blood, plasma, saliva, sweat, urine, tears, cerebrospinal fluid, abdominal dropsy, and amniotic fluid; organs such as liver, heart, brain, bone, hair, skin, nail, muscle, and nervous tissue, and extracts of tissues, cells, etc.

### 4. Activated Protein C

The activated protein C used in the activity assay method for total protein S in a specimen may be used without special limitations, regardless of its origin (source) or preparation method.

For example, it may include the one derived from mammals such as human, bovine and swine. Moreover, it may include the one purified from body fluids such as plasma, organs, or the like and prepared, or the ones prepared by genetic engineering operation, cell engineering operation, cell culturing, or the like.

In addition, in the assay reaction in the activity assay method for total protein S in a specimen, it is also possible to have activated protein C from protein C using a protein C activating substance, or the like, and use it as the activated protein C in the invention.

According to the activity assay method for total protein S in a specimen, when the specimen contains protein S, the activity of activated protein C increases in the presence of phospholipid and calcium ions, with the activated protein C contacts with the specimen.

Then, the activated protein C turns catalyzes a reaction that breaks down activated blood coagulation factor V in the presence of phospholipid and calcium ions.

Therefore, by the presence of protein S, the reaction that activated protein C breaks down activated blood coagulation factor V is promoted.

It is also possible to perform at once the contact of the specimen with the above-described activated protein C or the like, and the contact of activated protein C with activated blood coagulation factor V or the like.

In addition, it is also possible to perform the breakdown reaction of activated blood coagulation factor V by contacting the specimen with activated protein C as well as phospholipid, then incubating at least for not less than 1 min., preferably not less than 5 min., at room temperature or 37°C or the like, subsequently contacting with activated blood coagulation factor V and calcium ions, and incubating.

Typically, the concentration upon use of activated protein C is preferably 1 pM to 100 nM upon breaking down activated blood coagulation factor V in the presence of surfactant, phospholipid and calcium ions by contacting with activated blood coagulation factor V.

However, in order to obtain high measurement sensitivity, higher concentration of activated protein C is preferred, so that the concentration of activated protein C is more preferably 10 pM to 10 nM, and especially preferably 100 pM to 1 nM.

### 5. Surfactant

In the activity assay method for total protein S, it is preferred to have a surfactant present upon activity assay reaction for total protein S.

As the surfactant, it is possible to suitably have present one type or at least two types of surfactants, which include nonionic surfactants, ampholytic surfactants, anionic surfactants, or cationic surfactants.

Such surfactant may include, for example, nonionic surfactants such as sorbitan fatty acid ester, glycerin fatty acid ester, decaglycerin fatty acid ester, polyoxyethylene sorbitan fatty acid ester, polyoxyethylene glycerin fatty acid ester, polyethylene glycol fatty acid ester, polyoxyethylene alkyl ether, polyoxyethylene phytosterol, phytostanol, polyoxyethylene polyoxypropylene alkyl ether, polyoxyethylene alkyl phenyl ether, polyoxyethylene castor oil, hydrogenated castor oil, and polyoxyethylene lanolin; ampholytic surfactants such as acetate betaine; or anionic surfactants such as polyoxyethylene alkyl ether sulfate or polyoxyethylene alkyl ether acetate.

Here, when the surfactant is a nonionic surfactant, the one having HLB (hydrophile-lipophile balance) in the range of 10 to 20 is preferred, the one having in the range of 12 to 18 is more preferred, and the one having in the range of 13 to 16 is especially preferred.

As the surfactant, the nonionic surfactant may be preferably Triton X-100 [polyoxyethylene (n = 9, 10) p-t-octylphenyl ether, HLB: 13.5], Triton X-114 [polyoxyethylene (n = 7, 8) p-t-octylphenyl ether, HLB: 12.4], NP-10 [polyoxyethylene (n = 10) nonylphenyl ether, HLB: 16.5], NP-11 [polyoxyethylene (n = 11) nonylphenyl ether], NP-12 [polyoxyethylene (n = 12) nonylphenyl ether], NP-13 [polyoxyethylene (n = 13) nonylphenyl ether], NP-15 [polyoxyethylene (n = 15) nonylphenyl ether, HLB: 18.0], BT-9 [polyoxyethylene (n = 9) secondary alkyl ether, HLB: 13.5], BT-12 [polyoxyethylene (n = 12) secondary alkyl ether, HLB: 14.5], or the like.

Furthermore, the ampholytic surfactant may be preferably AM-301 [lauryl dimethyl amino acetate betaine aqueous solution], or the like.

Moreover, the anionic surfactant may be preferably sarcosinate LN [sodium lauroyl sarcosinate], or the like.

Furthermore, the cationic surfactant may be preferably CA-2350 [cetyltrimethylammonium chloride] or the like.

Here, the concentration of the surfactant present upon the reaction for measuring total protein S activity is not especially limited, but preferably 0.00001 to 5% (W/V), more preferably 0.0001 to 1% (W/V), even more preferably 0.001 to 0.1% (W/V), and especially preferably 0.005 to 0.05% (W/V).

In case of the nonionic surfactant, it is very preferably 0.001 to 0.01% (W/V); in case of the ampholytic surfactant, it is very preferably 0.001 to 0.01% (W/V); in case of the anionic surfactant, it is very preferably 0.001 to 0.1% (W/V); and in case of the cationic surfactant, it is very preferably 0.00001 to 0.001% (W/V).

Here, in the activity assay method for total protein S, upon the reaction, in which the activity of the activated protein C increases by protein S contained in the specimen, and upon the reaction, in which activated blood coagulation factor V is broken down by the activity-enhanced protein C, it is preferred to have the surfactant present for the activity assay of total protein S in a specimen.

### 6. Phospholipid

### (1) General

In the activity assay method for total protein S, phospholipid is present upon the reaction for measuring the total protein S activity.

The phospholipid can be used without specific limitations, regardless of the origin (source) or preparation method.

For example, it may include the ones derived from mammals such as human, bovine, or swine, the ones derived from other animals, the ones derived from plants, the ones derived from microorganisms, and artificially synthesized ones.

Upon the reaction for measuring total protein S activity, the concentration of the phospholipid to be present is normally, preferably 0.03 µM to 100 µM, more preferably 0.3 µM to 50 µM, and especially preferably 3 µM to 30 µM.

Such phospholipid may include, for example, phosphatidylcholine, phosphatidylserine, phosphatidylethanolamine, phosphatidylinositol, glycerophospholipid such as phosphatidylglycerol or diphosphatidylglycerol, and sphingophospholipid such as sphingomyelin.

Here, upon the reaction for measuring total protein S activity, as the phospholipid, phospholipid is composed of phosphatidylcholine, phosphatidylserine, and phosphatidylethanolamine present for activity assay for total protein S in a specimen.

In addition, in case of having present the phospholipid composed of phosphatidylcholine, phosphatidylserine and phosphatidylethanolamine, the composition of those three types of phospholipids is preferably such that the composition ratio of phosphatidylethanolamine is not less than 10% (W/V) and the composition ratio of phosphatidylserine is not less than 20% (W/V).

Especially, it is preferred that the composition ratio of phosphatidylethanolamine is not less than 30% (W/V) and the composition ratio of phosphatidylserine is not less than 30% (W/V).

### (2) Phospholipid to Use in Catalyzed Reaction by Activated protein C

According to the activity assay method for total protein S, upon the reaction, in which the activity of activated protein C increases by protein S contained in a specimen, and upon the breakdown reaction of activated blood coagulation factor V by the activated protein C, phospholipid is present, which is necessary for the activity enhancing reaction of the activated protein C and the breakdown reaction of activated blood coagulation factor V.

Phospholipid can be used without specific limitations, regardless of the origin (source) or preparation method.

For example, it may include the ones derived from mammals such as human, bovine or swine, the ones derived from other animals, the ones derived from plants, the ones derived from microorganisms, and artificially synthesized ones.

The concentration of phospholipid to be present upon the activity enhancing reaction of activated protein C and upon the breakdown reaction of activated blood coagulation factor is normally, preferably 0.1 µM to 100 µM, more preferably 1 µM to 50 µM, and especially preferably 10 µM to 30 µM.

Such phospholipid may include, for example, phosphatidylcholine, phosphatidylserine, phosphatidylethanolamine, phosphatidylinositol, glycerophospholipid such as phosphatidylglycerol or diphosphatidylglycerol, and sphingophospholipid such as sphingomyelin.

Here, as for the phospholipid, upon the above-described activity enhancing reaction of activated protein C and upon the breakdown reaction of activated blood coagulation factor V, it is preferred to have present phospholipid composed of phosphatidylcholine, phosphatidylserine, and phosphatidylethanolamine, for measuring the activity of total protein S in a specimen.

In addition, when the phospholipid composed phosphatidylcholine, phosphatidylserine and phosphatidylethanolamine is present, in the composition of those three types of phospholipids, it is preferred that the composition ratio of the phosphatidylethanolamine is not less than 10% (W/V), and the composition ratio of the phosphatidylserine is not less than 20% (W/V).

Especially, it is preferred that the composition ratio of phosphatidylethanolamine is not less than 30% (W/V), and the composition ratio of phosphatidylserine is not less than 30% (W/V).

### (3) Phospholipid to Use in Catalyzed Reaction by Activated Blood Coagulation Factor X

According to the activity assay method for total protein S, upon reaction that produces thrombin from prothrombin, which is catalyzed by activated blood coagulation factor X in the presence of activated blood coagulation factor V, necessary phospholipid is to be present in the reaction.

Phospholipid can be used without specific limitations, regardless of the origin (source) or preparation method.

For example, it may include the ones derived from mammals such as human, bovine or swine, the ones derived from other animals, the ones derived from plants, the ones derived from microorganisms, and artificially synthesized ones.

The concentration of phospholipid to be present in the reaction that produces thrombin from prothrombin is normally, preferably in the range of 0.1 µM to 100 µM, more preferably 0.5 µM to 50 µM, and especially preferably 1 µM to 10 µM.

Such phospholipid may include, for example, phosphatidylcholine, phosphatidylserine, phosphatidylethanolamine, phosphatidylinositol, glycerophospholipid such as phosphatidylglycerol or diphosphatidylglycerol, and sphingophospholipid such as sphingomyelin.

Here, upon the above-described reaction that produces thrombin from prothrombin, as the phospholipid, it is preferred to have present phospholipid composed of phosphatidylcholine and phosphatidylserine, or phosphatidylcholine, phosphatidylserine and phosphatidylethanolamine, since the effect of promoting the above-described reaction is higher.

In addition, when the phospholipid composed of phosphatidylcholine and phosphatidylserine is to be present, as for the composition of those two types of phospholipids, it is preferred that the composition ratio of phosphatidylcholine is 85% (W/V) to 50% (W/V) and the composition ratio of the phosphatidylserine is 15% (W/V) to 50% (W/V).

Especially, it is preferred that the composition ratio of the phosphatidylcholine is 80% (W/V) to 70% (W/V) and the composition ratio of the phosphatidylserine is 20% (W/V) to 30% (W/V).

Alternatively, when the phospholipid composed of phosphatidylcholine, phosphatidylserine and phosphatidylethanolamine is to be present, for the composition of those three types of phospholipids, it is preferred that the composition ratio of the phosphatidylethanolamine is not less than 10% (W/V) and the composition ratio of phosphatidylserine is not less than 20% (W/V).

Especially, it is preferred that the composition ratio of phosphatidylethanolamine is not less than 30% (W/V) and the composition ratio of phosphatidylserine is not less than 30% (W/V).

Here, as the phospholipid to be present upon the reaction to produce thrombin from prothrombin, it is possible to use the phospholipid as is, which was used upon the above-described activity enhancing reaction of activated protein C and upon the breakdown reaction of the activated blood coagulation factor V.

However, as described above, since suitable composition of phospholipid varies depending on the respective reactions, it is preferred to use having the phospholipid, which has suitable composition for the respective reaction, present upon the reaction.

Here, upon the reaction that produces thrombin from prothrombin, even if the previously added phospholipid having a composition suitable for the above-described activity enhancing reaction of the activated protein C and the breakdown reaction of the activated blood coagulation factor V is co-present with the phospholipid having a composition suitable for the reaction that produces thrombin from prothrombin, there is no problem at all in the activity assay method and the activity assay reagent for total protein S in a specimen of the invention.

### 7. Calcium Ions

In the activity assay method for total protein S in a specimen, upon the activity enhancing reaction of the activated protein C by protein S contained in the specimen, upon the breakdown reaction of the activated blood coagulation factor V by activated protein C, and upon the reaction that produces thrombin from prothrombin by activated blood coagulation factor X and the activated blood coagulation factor V, calcium ions is to be present.

Such calcium ions can be, of course, calcium ions themselves, or can be any without specific limitations as long as it is a compound that contains calcium ion, such as calcium salt.

The compounds containing calcium ions may include, for example, calcium fluoride, calcium chloride, calcium bromide, calcium iodide, calcium sulfate, calcium nitrate, calcium acetate, calcium lactate or calcium cyanide.

The concentration upon using the calcium ion may be normally, upon the activity enhancing reaction of activated protein C by protein S contained in the specimen, upon the breakdown reaction of activated blood coagulation factor V by activated protein C, and upon the reaction that produces thrombin from prothrombin by activated blood coagulation factor X and activated blood coagulation factor V, preferably 0.1 mM to 100 mM, and especially preferably 1 mM to 10 mM.

### 8. Activated Blood Coagulation Factor V

The activated blood coagulation factor V (Factor Va; FVa) to use in the activity assay method for total protein S in a specimen can be used without special limitations, regardless of its origin (source) or preparation method.

For example, it may include the ones derived from mammals such as human, bovine and swine.

In addition, it may include the ones purified and prepared from body fluids such as plasma or organs, or the ones prepared by genetic engineering operations, cell engineering operations or cell culturing operations or the like.

According to the activity assay method for total protein S in a specimen, the activated blood coagulation factor V is to be broken down by activated protein C in the presence of a surfactant, phospholipid and calcium ions.

Then, when the specimen contains protein S, by the presence of the protein S, the activity of activated protein C increases, and the breakdown reaction is promoted.

In addition, the activated blood coagulation factor V promotes the reaction that produces thrombin from prothrombin, which is catalyzed by activated blood coagulation factor X in the presence of phospholipid and calcium ions.

Therefore, when the specimen contains protein S, the activity of activated protein C increases, the breakdown reaction of activated blood coagulation factor V is promoted, and the existing amount (concentration) of activated blood coagulation factor V decreases.

Accordingly, since the effect of activated blood coagulation factor V to promote the reaction that produces thrombin from prothrombin, which is catalyzed by the activated blood coagulation factor X, becomes less, the above-described reaction that produces thrombin from prothrombin is suppressed.

It may be possible to perform at the same time the above-described contact of activated blood coagulation factor V with activated protein C or the like, and the contact of activated blood coagulation factor V, activated blood coagulation factor X, prothrombin, and the like.

However, it is preferred to contact activated blood coagulation factor V with a surfactant, phospholipid and calcium ions as well as with activated protein C, incubate at least for not less than 1 min., preferably not less than 5 min., at room temperature or 37°C or the like, subsequently contact with activated blood coagulation factor X, prothrombin, and the like, and incubate so as to perform the reaction to produce thrombin from prothrombin.

The concentration upon use of activated blood coagulation factor V may be normally, preferably 0.5 pM to 5 nM, upon contacting with the activated protein C and performing the breakdown reaction of the activated blood coagulation factor V in the presence of a surfactant, phospholipid and calcium ions.

However, when the concentration of activated blood coagulation factor V is high, the blood coagulation reaction by a component (factor) or the like from the specimen could progress, thereby fibrins may be deposited or significant color development may occur, so that it may be impossible to perform accurate measurement; therefore the concentration of the above-described activated blood coagulation factor V may be more preferably 5 pM to 500 pM, and especially preferably 20 pM to 200 pM.

### 9. Activated Blood Coagulation Factor X

The activated blood coagulation factor X (Factor Xa; FXa) used in the activity assay method for total protein S in a specimen can be used without specific limitations, regardless of its origin (source) or preparation method.

For example, it may include the ones derived from mammals such as human, bovine or swine.

In addition, it may include the ones purified and prepared from body fluids such as plasma or organs, or the ones prepared by genetic engineering operations, cell engineering operations or cell culturing operations or the like.

According to the activity assay method for total protein S in a specimen, the activated blood coagulation factor X catalyzes the reaction that produces thrombin from prothrombin in the presence of phospholipid and calcium ions.

The reaction that produces thrombin from prothrombin by activated blood coagulation factor X is promoted by the presence of activated blood coagulation factor V.

Accordingly, as described above, when the specimen contains protein S, the activity of activated protein C increases, thereby the breakdown reaction of activated blood coagulation factor V is promoted, so that the effect of activated blood coagulation factor V to promote the reaction to produce thrombin, which is catalyzed by activated blood coagulation factor X, becomes less, and the above-described reaction that produces thrombin is suppressed.

The concentration upon using the activated blood coagulation factor X may be normally, preferably 0.1 pM to 300 pM upon the production of thrombin from prothrombin by contacting activated blood coagulation factor V, prothrombin, and the like in the presence of phospholipid and calcium ions.

However, when the concentration of the activated blood coagulation factor X is high, similarly to the aforementioned case of activated blood coagulation factor V, the blood coagulation reaction by a component (factor) from the specimen may progress, and fibrin may be deposited or significant color development may occur, so that it is impossible to perform accurate measurement; therefore the concentration of the above-described activated blood coagulation factor X may be more preferably 1 pM to 100 pM, and especially preferably 10 pM to 50 pM.

### 10. Prothrombin

The prothrombin used in activity assay method for total protein S in a specimen can be used without specific limitations, regardless of its origin (source) or preparation method.

For example, it may include the ones derived from mammals such as human, bovine or swine.

In addition, it may include the ones purified and prepared from body fluid such as plasma or organs, or the ones prepared by genetic engineering operations, cell engineering operations or cell culturing operations.

According to the activity assay method for total protein S in a specimen, prothrombin serves as a substrate in the reaction catalyzed by activated blood coagulation factor X in the presence of phospholipid and calcium ions, and turns into thrombin.

The reaction that produces thrombin from prothrombin by activated blood coagulation factor X is promoted by the presence of activated blood coagulation factor V.

Therefore, as described above, when the specimen contains protein S, the activity of activated protein C increases, thereby the breakdown reaction of the activated blood coagulation factor V is promoted, so that the effect of activated blood coagulation factor V to promote the reaction that produces thrombin from prothrombin, which is catalyzed by the activated blood coagulation factor X, is less; therefore the above-described reaction that produces thrombin from prothrombin is suppressed and the amount (concentration) of thrombin is reduced.

The concentration upon using prothrombin may be normally, upon contacting activated blood coagulation factor V and activated blood coagulation factor X, and producing thrombin from prothrombin in the presence of phospholipid and calcium ions, preferably 1 nM to 50 µM, more preferably 50 nM to 5 µM, and especially preferably 100 nM to 1 µM.

### 11. Substrate of Thrombin

A substrate of thrombin used in the activity assay method for total protein S in a specimen can be any without specific limitations, as long as it produces some signals by receiving a catalyst action as a protease of thrombin (as a substrate of thrombin), or it produces some signals by further continuing another reaction in addition to the catalyzed reaction by thrombin.

The production of some signals means that it is possible to detect production or change of signals optically, electrically, magnetically, or by other energy, by receiving a catalytic action of thrombin.

For example, it may include the one that absorbance, transmittance, or fluorescence intensity changes, light absorption curve changes, or that light is emitted, by receiving a catalyst action of thrombin.

An example of this may include, peptide or protein bound with a compound, such that absorbance, transmittance or fluorescence intensity, or light absorption curve changes when freed; peptide or protein bound with a compound, such that light is emitted when freed; and the like, and a substance that the above-described compound is freed from the above-described peptide or protein by a catalytic action of thrombin.

In case of such substance, when it is subjected to a catalytic action of thrombin and has the compound freed, it may be detected as a change in absorbance, transmittance or fluorescence intensity or light absorption curve, light emission, or the like, the catalytic action of thrombin, i.e. enzyme activity of thrombin, may be determined by measuring the amount of produced signals (change in absorbance, transmittance, fluorescence intensity, light absorption curve, light emission, or the like).

Such substance may include, for example, "H-D-phenylalanyl-L-pipecolyl-L-arginyl-p-nitroanilide·dichloride" [Testzyme® chromogenic substrate S-2238] (manufactured by: Chromogenix-Instrumentation Laboratory Co. [Italy], Sold by: Sekisui Medical Co., Ltd. [Japan]), "H-D-hexahydrotyrosyl-L-alanyl-L-arginyl-p-nitroanilide.diacetate" [SPECTROZYME® TH] (American Diagnostica Co., Cosmo Bio Co., Ltd.), "benzoyl-phenylalanyl-vanillyl-arginyl-p-nitroanilide chloride" [Thrombin Substrate I, Colorimetric] (CALBIOCHEM Co.·Cosmo Bio Co., Ltd.), "tosyl-glycyl-prolyl-arginyl-p-nitroanilide" [CHROMOZYME TH](Pentapharm Co.), "H-D-phenylalanyl-prolyl-arginyl-3-carboxy-4-hydroxy-aniline" (Daiichi Sankyo Co., Ltd.), "benzoyl-phenylalanyl-vanillyl-arginyl-AMC chloride" [Thrombin Substrate III, Fluorogenic] (Calbiochem Corp., Cosmo Bio Co., Ltd.), "t-butoxycarbonyl-asparagyl(o-benzyl)-prolyl-arginyl-MCA" (Peptide Institute, Inc.), or "t-butoxycarbonyl-vanillyl-prolyl-arginyl-MCA" (Peptide Institute, Inc.). Here, especially "H-D-phenylalanyl-L-pipecolyl-L-arginyl-p-nitroanilide·dichloride" is preferred.

It may be possible to perform at the same time or as separate steps the above-described contact of activated blood coagulation factor V, activated blood coagulation factor X, prothrombin, and the like, and the contact of the resultant thrombin with the substrate of thrombin.

Here, in either case, the substrate of thrombin is contacted with the resultant thrombin, and incubated at least for not less than 1 min., preferably not less than 5 min. at room temperature or 37°C or the like, so as to have signals produced from the substrate of thrombin.

The concentration upon using the substrate of thrombin may be normally, when the substrate of thrombin is subjected to the catalyst action of thrombin, preferably 5 µM to 100 mM, and especially preferably 50 µM to 10 mM.

### 12. Measurement of Amount of Signals

The substrate of thrombin in the activity assay method for total protein S in a specimen is subjected to a catalytic action of thrombin produced from prothrombin, and thereby produces signals,.

In the activity assay method for total protein S in a specimen, the amount of signals produced from the substrate of thrombin is measured.

Here, when the specimen contains protein S, the amount of signals is the amount of signals, production of which is restricted according to the activity of total protein S in the specimen.

The measurement of the amount of produced signals (the amount of signals restricted from production) can be suitably performed according to the signals.

For example, when the signals are changes in absorbance, transmittance, fluorescence intensity, light absorption curve, light emission, or the like, the measurement is performed by measuring the absorbance, transmittance, fluorescence intensity, or fluorescence intensity, or the like.

More specifically, when the substrate of thrombin is a substance, in which p-nitroaniline is bound to peptide, such as the above-described "H-D-phenylalanyl-L-pipecolyl-L-arginyl-p-nitroanilide·dichloride", "H-D-hexahydrotyrosyl-L-alanyl-L-arginyl-p-nitroanilide·diacetate", "benzoyl-phenylalanyl-vanillyl-arginyl-p-nitroanilide·chloride", or "tosyl-glycyl-prolyl-arginyl-p-nitroanilide", the measurement is performed by measuring absorbance at or near the wavelength of 405 nm for the light absorbance near the wavelength of 405 nm by p-nitroaniline freed by the catalytic action of thrombin.

In this case, the measurement of absorbance can be a single-wavelength measurement conducted only at a dominant wavelength or two-wavelength measurement measured at a dominant wavelength and a complimentary wavelength.

Moreover, the absorbance measurement can be an end-point method or a rate method.

### 13. Activity of Total Protein S Contained in a Specimen

In the activity assay method for total protein S in a specimen, when the specimen contains protein S, the activity of activated protein C increases, the breakdown reaction of activated blood coagulation factor V is promoted, and the amount of exiting (concentration of) activated blood coagulation factor V becomes less.

Accordingly, since the promoting effect of the activated blood coagulation factor V in the reaction that produces thrombin from prothrombin, which is catalyzed by activated blood coagulation factor X, is less, the above-described reaction that produces thrombin from prothrombin is restricted.

Therefore, since the production of thrombin decreases, the reaction that produces signals from a substrate of thrombin, which is catalyzed by thrombin, is also restricted, and the amount of signals to be produced is suppressed.

In other words, according to the activity of total protein S contained in the specimen, the degree of restriction on the signal production increases.

Accordingly, in the activity assay method for total protein S in a specimen, by measuring the amount of signals produced by contacting a specimen with activated protein C and the like so as to perform the above-described reaction, i.e. measuring the amount of signals restricted from production, the activity of total protein S contained in the specimen is obtained.

After measuring the amount of signals restricted from production, the activity of total protein S contained in the specimen may be suitably obtained, but, for example, can be performed as follows.

For at least one of specimens with known activity of total protein S and a specimen, total protein S activity of which is known as "zero" (a saline solution or purified water, or the like.), the above-described measurement procedure is performed, and the amount of produced signals (the amount of signals restricted from production) is determined.

Then, the relation between the amount of produced signals (the amount of signals restricted from production) and the activity of total protein S contained in the specimen is expressed as a numerical equation, graph, or the like, and a calibration curve is made.

The calibration curve, in other words, shows the relation between the degree of restriction on the signal production and the activity of total protein S contained in a specimen.

Next, similarly to the above, the measurement procedure is performed for a specimen with unknown total protein S activity, and the amount of produced signals (the amount of signals restricted from production) is determined.

Fitting the signal amount to the above-described calibration curve, and corresponding activity of total protein S is determined.

As such, from the amount of produced signals (the amount of signals restricted from production) in the specimen with unknown total protein S activity, it is possible to obtain activity of total protein S in the specimen.

Here, the above-described calibration curve shows the relation between the signal amount (the amount of signals restricted from production), i.e., the degree of restriction on the signal production, and the activity of total protein S contained in a specimen, but the signal amount (the amount of signals restricted from production) in the calibration curve can be the measured signal amount itself, but can be a numerical value calculated based on the signal amount.

In other words, the above-described calibration curve can be the one that shows the relation between a numerical value calculated based on the measured signal amount and the activity of total protein S contained in the specimen.

Here, even in this case, the numerical value calculated based on the value of the measured signal amount corresponds to the amount of signals restricted from production.

Such numerical value calculated based on the value of the measured signal amount may include, for example, a numerical value of change in the measured signal amount per unit time, a numerical value calculated by a numerical value calculated by first derivation of a value of the measured signal amount by time, or a value calculated by second derivation.

For example, it may include the change per 1 min. in the absorbance obtained by measurement, or speed of the absorbance change obtained by first derivation of the absorbance obtained by measurement by time, or acceleration of the absorbance change obtained by second derivative of the absorbance obtained by measurement by time, or the like.

### 14. Protein

In the activity assay method for total protein S in a specimen, preferably, albumin such as human serum albumin (HSA), bovine serum albumin (BSA) or ovalbumin, or protein such as casein and gelatin is present upon the above-described activity assay reaction.

The concentration of the protein to be present is normally, preferably 0.1 µM to 1 mM.

### 15. Salts

In the activity assay method for total protein S in a specimen, salts such as salt of halogen element and alkali metal or salt of halogen element and alkali earth metal is preferably present upon the above-described activity assay reaction.

Such salt of halogen element and alkali metal may include, for example, sodium chloride, potassium chloride, sodium fluoride, potassium fluoride, sodium bromide, or potassium bromide.

In addition, the salt of halogen element and alkali earth metal may include, for example, magnesium chloride, magnesium fluoride, or magnesium bromide.

The concentration of the salt to be present is normally, preferably 5 mM to 1 M, and especially preferably 50 mM to 250 mM.

### 16. Diluent

In the activity assay method for total protein S in a specimen, it is preferred to perform the above-described activity assay reaction after diluting the specimen with a diluent, for the activity assay of total protein S in the specimen.

The pH of the diluent may not be specifically limited, but preferably in the range of pH 6.0 to pH 10.0 (20°C), and more preferably in the range of pH 6.5 to pH 8.5 (20°C).

Here, in the diluent, for a purpose to keep the pH within the above-described pH range, it is preferred to have a buffer suitably having a buffering effect in the above-described pH range present or contained.

In addition, the diluent can suitably contain a component such as protein, salt, surfactant, preservative, stabilizer, activator, or saccharide, as necessary.

The diluent may include, for example, water, a saline solution, a phosphate buffered saline solution, or a buffer solution.

The dilution rate upon diluting a specimen with the diluent is not specifically limited, but for the activity assay of total protein S in a specimen, it is preferably 2 times to 60 times, more preferably 5 times to 40 times, and especially preferably 10 times to 20 times.

### 17. pH

In the activity assay method for total protein S in a specimen, the above-described activity assay reaction is preferably performed in the range of pH 6.0 to pH 10.0 (20°C), and especially preferably in the range of pH 6.5 to pH 8.5 (20°C) .

Here, in the activity assay method for total protein S in a specimen, in order to keep the above-described pH within the range in the activity assay reaction, it is preferred to have a buffer suitably present, which has a buffering effect in the above-described pH range.

### 18. Other components

In the activity assay method for total protein S in a specimen, it is possible to suitably have a component other than the above-described components, such as a preservative, stabilizer, activator, or saccharide, present upon the above-described activity assay reaction as necessary.

### 19. Assay Method

According to the activity assay method for total protein S in a specimen, the activity assay of total protein S in a specimen can be performed by manual procedure or by using a device such as an automated analyzer.

### 20. Measurement Steps

In the activity assay method for total protein S in a specimen, the above fully described activity assay reaction can be done by performing all the reactions in one step by contacting all the components with specimen at once (1-step method), or can be performed by dividing the components and performing the contact in several steps so as to divide the activity assay reaction into several steps (multi-step method).

In case of dividing into a plurality of steps, there is no specific limitation, but for example, it is possible to perform as follows:

### (1) Two-Step Method-1

(a) Contact a specimen, activated protein C, surfactant, phospholipid, calcium ion and activated blood coagulation factor V.
(b) Next, contact the above-described mixture (a) with activated blood coagulation factor X, prothrombin and a substrate of thrombin.

### (2) Two-Step Method-2

(a) Contact a specimen, activated protein C, surfactant, phospholipid and calcium ion.
(b) Next, contact the above-described mixture (a) with activated blood coagulation factor V, activated blood coagulation factor X, prothrombin, a substrate of thrombin, and phospholipid.

### (3) Three-Step Method

(a) Contact a specimen, activated protein C, a surfactant, phospholipid, and calcium ion.
(b) Next, contact the above-described mixture (a) with activated blood coagulation factor V.
(c) Furthermore, contact the above-described mixture (b) with activated blood coagulation factor X, prothrombin, and a substrate of thrombin.

### III. Method of Measuring Protein Content of Total protein S in a Specimen

As described above, according to the invention, the method (assay method) of and the reagent (assay reagent) for measuring a protein content of total protein S are not specifically limited, and can be any as long as they are the method and the reagent, whereby it is possible to conduct the assay of protein content of total protein S; hereunder, an example of the method of measuring protein content of total protein S in a specimen will be described.

### 1. General

In a method of measuring protein content of total protein S in a specimen that includes the steps of contacting the specimen and carrier particles, to which an antibody for protein S is fixed, and measuring coagulate produced from an antigen-antibody reaction of the above-described antibody with protein S contained in the specimen so as to measure the protein content of total protein S in the specimen, it is preferred to have C4b-binding protein present upon the above-described antigen-antibody reaction.

Here, in the protein content assay method for total protein S in a specimen, the carrier particles are preferably latex particles.

In addition, according to the method of measuring protein content of total protein S in a specimen, the assay of protein content of total protein S in a specimen is preferably performed by a method containing the following steps (a) and (b) :
(a) A step of mixing a specimen and C4b-binding protein and contacting, so as to form in the mixed solution a complex of free protein S contained in the above-described specimen and C4b-binding protein.
(b) A step of contacting the above-described mixed solution with carrier particles, to which antibody to protein S is fixed, and measuring coagulates produced from an antigen-antibody reaction between the above-described antibody and the complex of protein S and C4b-binding protein.

### 2. Antibody to Protein S

According to the method of measuring protein content of total protein S in a specimen, the antibody to protein S means antibody that can bind to protein S (anti-protein S antibody).

Such anti-protein S antibody may include, for example, antiserum including monoclonal antibody (that is capable of binding to protein S), polyclonal antibody, polyclonal antibody, chimera antibody, humanized antibody or single-strand antibody (scFv), and fragments of those antibodies [Fab, F(ab')2, Fab', Fv, sFv, dsFv, etc.].

In addition, it is also possible to use a derivative, in which protein or low molecular compound is bound to the above-described antibody or antibody fragments.

Here, the origin of the anti-protein S antibody is not specifically limited, but may include, for example, mammals (mouse, rabbit, rat, ovine, caprine, equine, etc.), or birds (chicken, quail, pheasant, ostrich, duck, etc.).

### 3. Carrier Particles

According to the method of measuring protein content of total protein S in a specimen, the carrier particles can be used without specific limitations, as long as it can fix the above-described anti-protein S antibody.

More specifically, it can be any as long as it is carrier particles that are used for the assay reagent and the assay method for measuring protein content of total protein S in a specimen, using the antigen-antibody reaction between protein S and anti-protein S antibody.

A material of the carrier particles is not specifically limited and may include, for example, polystyrene, styrene-styrene sulfonate copolymer, acrylonitrile-butadienestyrene copolymer, vinyl chloride-acrylate ester copolymer, vinyl acetate-acrylic acid copolymer, polyacrolein, styrene-methacrylic acid copolymer, styrene-glycidyl(metha)acrylic acid copolymer, styrene-butadiene copolymer, methacrylic acid polymer, acrylic acid polymer, gelatin, silica, alumina, carbon black, metal compounds, metal, ceramics, or magnetic substances.

Furthermore, the carrier particles may include, for example, particles such as latex particles, metal colloidal particles, liposome, microcapsules, red blood cells, or the like.

In addition, the carrier particles are preferably latex particles.

According to the method of measuring the protein content of total protein S in a specimen, fixation of anti-protein S antibody on the carrier particles may be done by a known method, such as a physical adsorption method, chemical bonding method, combination of those, etc.

In case of the physical adsorption method, it can be done according to a known method, by mixing and contacting the anti-protein S antibody with the carrier particles in a solution such as a buffer solution, or contacting the anti-protein S antibody dissolved in a buffer solution or the like with the carrier particles, or the like.

In addition, in case of performing by a chemically binding method, according to a known method described in "Japanese Society of Laboratory Medicine Edition "Clinical Pathology, Extra Edition Special Issue No. 53 Immuno Assay for Laboratory Tests-Techniques and Applications-", Clinical Pathology Publication Society, issued in 1983; Japanese Biochemical Society Ed. "New Biochemistry Experiment Courses 1, Protein IV", Tokyo Kagaku Dojin, issued in 1991, or the like, it is possible to perform by mixing and contacting the anti-protein S antibody and the carrier particles with a binary crosslinking reagent such as glutaraldehyde, carbodiimide, imide ester, or maleimide, so as to cause the reaction between the respective amino groups, carboxyl groups, thiol groups, aldehyde groups or hydroxyl groups, or the like of anti-protein S antibody and carrier particles with the above-described binary crosslinking reagent, or by other method.

Furthermore, when treatment for restricting natural coagulation of the carrier particles, to which anti-protein S antibody is fixed, or nonspecific reaction, or the like is necessary, blocking treatment (masking treatment) of the carrier particles may be performed by a known method, such as contacting and coating the surfaces of the carrier particles, on which anti-protein S antibody is fixed, with protein such as bovine serum albumin (BSA), casein, gelatin, ovalbumin or their salts, a surfactant or skim milk powder, and the like.

Here, when the measurement of protein content of total protein S contained in a specimen is performed by nephelometry such as latex immunonephelometry, the size (particle diameter) of the carrier particles such as latex particles is not specificaly limited.

However, based on reasons such as the degree of producing coagulate (aggregate) of the carrier particles, to which anti-protein s antibody is fixed, with protein S contained in the specimen, the easiness to measure the produced coagulates, or the like, as for the size (particle diameter) of the carrier particles, the average diameter (average particle diameter) is preferably 0.01 µm to 10 µm, and more preferably 0.04 µm to 1 µm.

In addition, according to the method of measuring protein content of total protein S in a specimen, the carrier particles to use can be two or more types of carriers having different sizes (particle diameters), materials, shape, or the like.

Here, in case of measuring the protein content of total protein S in a specimen by nephelometry such as latex immunonephelometry, the optimum concentration of the carrier particles, to which anti-protein S antibody is fixed, may vary depending on conditions, such as distribution density of the above-described specifically bound substance on the carrier surface, the size (particle diameter) of the carrier particles, the mixing ratio of the specimen and the assay reagent, so that it cannot be specifically set.

However, normally, when the specimen and the assay reagent are mixed and the assay reaction occurs, in which an antigen antibody reaction occurs between anti-protein S antibody fixed to carrier particles and protein S contained in a specimen, it is typical to have the concentration of the carrier particles, to which anti-protein S antibody is fixed, at 0.005 to 1% (W/V) in the mixed reaction solution upon the assay reaction; and in this case, it is preferred to have the carrier particles, to which anti-protein S antibody is fixed, contained in the assay reagent, so as to be at such concentration in the mixed reaction solution,.

According to the method of measuring protein content of total protein S in a specimen, the carrier particles, to which anti-protein S antibody is fixed, may be co-present with protein such as bovine serum albumin (BSA), human serum albumin (HSA), casein, or their salts; metal ions such as calcium ions; salts such as calcium salts; saccharides; skim milk powder; animal serum such as normal rabbit serum; preservatives such as sodium azide or antibiotics; activators; reaction accelerator; sensitivity enhancers such as polyethylene glycol; nonspecific reaction inhibitor; or one or two or more types of surfactants such as nonionic surfactant, ampholytic surfactant or anionic surfactant.

In addition, the concentration of the above-described respective substance to be co-present may not be specifically limited, but preferably 0.001 to 10% (W/V), and especially preferably 0.01 to 5% (W/V).

### 4. C4b-binding Protein

According to the method of measuring protein content of total protein S in a specimen, it is preferred to have C4b-binding protein present. Here, the C4b-binding protein means macromolecular glycoprotein produced in liver cells or macrophages, and specifically makes a one-to-one binding with protein S to form a complex.

In addition, the C4b-binding protein to use in the method of measuring protein content of total protein S in a specimen can be used without specific limitations, regardless of its origin (source) or preparation method.

For example, it may include the ones from mammals, such as human, bovine or swine. In addition, it may also include the ones purified and prepared from body fluid such as plasma or organs, or the ones prepared by genetic engineering operations, cell engineering operations or cell culturing operations, or the like.

According to the method of measuring protein content of total protein S in a specimen, the concentration of the above-described C4b-binding protein to be present in the assay reagent for protein content of total protein S may be preferably set such that the ratio of C4b-binding protein and free protein S contained in the specimen is at least 0.9 (C4b-binding protein/free protein S ≥ 0.9) in the assay reaction solution after mixing the specimen and the assay reagent.

Here, the method of having the above-described C4b-binding protein present in the assay reagent for protein content of total protein S can be any, as long as it is possible to have the c4b-binding protein present in the above-described assay reagent for protein content of total protein S upon the assay reaction to perform an antigen-antibody reaction between the anti-protein S antibody fixed to carrier particles and the protein S contained in the specimen.

For example, it is possible to prepare a reagent, in which the above-described C4b-binding protein is contained in a buffer solution, and mix with a reagent containing carrier particles, to which anti-protein S antibody is fixed, so as to have the C4b-binding protein present upon the assay reaction to perform the antigen antibody reaction between the anti-protein S antibody that is fixed to the carrier particles and protein S contained in the specimen.

Here, normally in a specimen of a healthy individual, both "a complex of protein S and C4b-binding protein (bound form)" and "free protein S (free form)" are present, but according to the invention, by mixing and contacting the C4b-binding protein with a specimen, free protein S contained in the specimen forms a complex with the C4b-binding protein.

More specifically, any protein S in a specimen becomes the "complex of protein S and C4b-binding protein (bound form)".

As such, according to the method of measuring protein content of total protein S in a specimen, it is possible to measure the protein content of any protein S [complex of protein S and C4b-binding protein (bound form) and free protein S (free form)], i.e., protein content of total protein S.

### 5. Specimen

According to the method of measuring protein content of total protein S in a specimen, the specimen means the one, in which protein S may exist, and which is provided for determining the presence or the content (concentration) of protein S.

Such specimen may include, for example, body fluids such as human or animal blood, serum, plasma, saliva, sweat, urine, tear, cerebrospinal fluid, amniotic fluid, and abdominal dropsy, or organs such as liver, heart, brain, bone, hair, skin, nail, muscle, nervous tissue, extracts from tissues or cells, or the like, which possibly contains protein S.

### 6. Assay Method

The assay method for protein content of total protein S in a specimen is the method of measuring protein content of total protein S in a specimen by measuring coagulates produced from the antigen-antibody reaction between "antibody to protein S" that is fixed to the carrier particles and "protein S" contained in the specimen, with C4b-binding protein is present upon occurrence of the antigen-antibody reaction.

Measurement operations in the method of measuring protein content of total protein S in a specimen can be performed according to known measurement operation.

The measurement can be performed by manual procedure, or by using a device such as analyzers.

In addition, the measurement can be done by a 1-step method (1-reagent method) or by a method of multiple operational steps such as 2-step method (2-reagent method).

Hereunder, detailed explanation will be provided using as an example a case of performing the measurement of protein content of total protein S in a specimen using an assay reagent for measuring protein content of total protein S in a specimen and latex immunonephelometry as measurement principle.
(1) First, as the assay reagent for protein content of total protein S in a specimen, prepare the following items:
   First reagent: Buffer solution that contains C4b-binding protein.
   Second reagent: Buffer solution that contains latex particles, to which antibody to protein S is fixed.
(2) Mix a certain amount of a specimen such as plasma and a certain amount of the above-described first reagent, and let it stand for a certain amount of time at a certain temperature.
   Here, the mixing ratio (amount ratio) of the specimen and the first reagent can be suitably selected.
   In addition, the temperature upon the above-described specimen standing may be preferably a certain temperature (e.g., 37°C) within the range of room temperature (1 to 30°C) or slightly warm temperatures (30 to 40°C).
   By mixing the specimen and the first reagent, free protein S contained in the specimen forms a complex with the C4b-binding protein.
   In other words, any protein S in the specimen becomes a "complex of protein S and C4b-binding (bound form).
(3) After a certain amount of time, into the mixed solution of the above-described specimen and the first reagent, add and mix a certain amount of the above-described second reagent so as to make a mixed reaction solution, and then let it stand for a certain amount of time under a certain temperature condition.
   Here, the amount of the second reagent to add may be suitably selected.
   In addition, the temperature upon the above-described standing may be a certain temperature (e.g., 37°C or the like) within the range of room temperature (1 to 30°C) or slightly warm temperatures (30 to 40°C).
   Moreover, the duration of the above-described standing may be preferably a certain period of not less than 1 min., but not more than 10 min., and more preferably a certain period of not less than 3 min., but not more than 5 min.
   By adding and mixing the second reagent in the mixed solution of the specimen and the first reagent, perform the antigen-antibody reaction (assay reaction) between anti-protein S antibody that is fixed to latex particles and the protein S in the specimen (complex of protein S and C4b-binding protein (bound form)).
   In addition, by the antigen-antibody reaction (assay reaction), cross-linking of "[anti-protein S antibody=latex particles=anti-protein S antibody]-[protein S]-[anti-protein S antibody=latex particles=anti-protein S antibody]..." is formed, and coagulates between latex particles, to which anti-protein S antibody is fixed, are formed.
(4) Moreover, in case of an analyzer, spectrophotometer, or the like, by irradiating light to a mixed reaction solution, and measuring decrease in intensity of transmitted beam (increase in absorbance) or increase in intensity of scattered light at a suitable wavelength, which are signals produced by the coagulates of latex particles, determine the amount of the above-described coagulates that are produced, i.e. protein content of total protein S in the specimen.
(5) Then, by comparing between "the measurement value obtained by performing the measurement on the specimen (decrease in intensity of transmitted beam (increase of absorbance) or a value of increase in intensity of scattered beam)" and "the measurement value (decrease in intensity of transmitted beam (increase of absorbance) or a value of increase in intensity of scattered beam) obtained by conducting the measurement on the above-described standard solution, and a standard substance such as standard serum (a specimen containing protein S with known concentration)", calculate protein content (concentration) of total protein S in the measured specimen.

### [Example]

Hereunder, the invention will be further described by examples. Here, the invention shall not be limited those examples.

### [Example 1] (Comparison between Total Protein S Activity and Protein Content of Total Protein S)

Comparison was made between the activity value of protein S obtained by the activity assay method and the activity assay reagent for total protein S in a specimen and the protein content of total protein S obtained by the assay method and the assay reagent for protein content of total protein S in a specimen according to latex nephelometry.

### 1. Measurement by the Assay Method and Assay Reagent for Total Protein S in a Specimen

### 1. Activity Assay Reagent for Total protein S

### (1) Diluent

Dissolving the following components in purified water so as to have the respectively indicated concentrations and adjusting pH to pH 8.0 (20°C), a diluent was prepared.

| | |
|---|---|
| Triton X-100(Wako Pure Chemical Industries, Ltd., Japan) | 0.060 % (W/V) |
| Bovine serum albumin (BSA) | 0.1% (W/V) |
| Sodium chloride | 0.1 M |
| Trisodium citrate | 10.6 mM |
| Tris(hydroxymethyl)aminomethane | 50 mM |

### (2) First Reagent

Dissolving the following components in purified water so as to have the respectively indicated concentrations and adjusting pH to pH 8.0 (20°C), a first reagent was prepared.

| | |
|---|---|
| Activated protein C (purified human activated protein C; Enzyme Research Laboratories, Inc., USA) | 397 pM |
| Surfactant | |
| Phospholipid | |
| Calcium chloride | 2.5mM |
| Bovine serum albumin | |
| (BSA; Sigma-Aldrich Co., USA) | 0.1% (W/V) |
| Sodium chloride | 0.1 M |
| Tris(hydroxymethyl)aminomethane | 50 mM |

Here, the surfactant was made to contain Triton X-100 (Wako Pure Chemical Industries, Ltd., Japan) in the concentration of 0.006% (W/V).

In addition, phospholipid was made to be contained in the concentration of 24 µM, with the phospholipid was prepared by respectively taking into a testing tube 0.4 mg phosphatidylserine (pig-brain phosphatidylserine; PS; Doosan Serdary Research Laboratories Co., South Korea), 0.4 mg phosphatidylcholine(pig-liver phosphatidylcholine; PC; Doosan Serdary Research Laboratories Co., South Korea), and 0.4 mg phosphatidylethanolamine (pig-liver phosphatidylethanolamine; PE; Doosan Serdary Research Laboratories Co., South Korea), then evaporating chloroform, the solvent, with an evaporator, thereafter adding distilled water and vigorously stirring for 1 min., and then performing sonication at 60°C for 10 min., and had the composition ratio of phosphatidylserine, phosphatidylcholine and phosphatidylethanolamine 1 : 1 : 1 (i.e., 33.33% (W/V) : 33.33% (W/V) : 33.33% (W/V)). [Here, phospholipid that has the composition ratio of the phosphatidylserine, phosphatidylcholine and phosphatidylethanolamine as 1 : 1 : 1 may be referred to as "phospholipid (PS : PC : PE = 1 : 1 : 1)" below.]

### (3) Second Reagent

Dissolving the following components so as to have the respectively-indicated concentrations and adjusting pH to pH 8.0 (20°C), a second reagent was prepared.

| | |
|---|---|
| Activated blood coagulation factor V (purified human activated blood coagulation factor V; Haematologic Technologies, Inc., USA) | 357 pM |
| Surfactant | |
| Phospholipid | |
| Calcium chloride | 2.5 mM |
| Bovine serum albumin | |
| (BSA; Sigma-Aldrich Co., USA) | 0.1% (W/V) |
| Sodium chloride | 0.1 M |
| Tris(hydroxymethyl)aminomethane | 50 mM |

Here, the surfactant was made to contain Triton X-100 (Wako Pure Chemical Industries, Ltd., Japan) in the concentration of 0.006% (W/V).

In addition, the phospholipid was made to contain the above-described phospholipid (PS : PC : PE = 1 : 1 : 1) in the concentration of 24 µM.

### (4) Third Reagent

Dissolving in purified water the following components so as to have the respectively-indicated concentrations and adjusting pH to pH 7.5 (20°C), a third reagent was prepared.

| | |
|---|---|
| Activated blood coagulation factor X (purified bovine activated blood coagulation factor X; New England Biolabs, Inc., USA) | 50 pM |
| Prothrombin (purified human prothrombin; Enzyme Research Laboratories, Inc., USA) | 738 nM |
| Testzyme® chromogenic substrate S-2238 (substrate of thrombin [chromogenic substrate of thrombin]; manufactured by: Chromogenix-Instrumentation Laboratory Co.[Italy], Sold by: Sekisui Medical Co., Ltd. [Japan] | 750 µM |
| Phospholipid | |
| Calcium chloride | 5.0 mM |
| Bovine serum albumin | |
| (BSA; Sigma-Aldrich Co., USA) | 0.1% (W/V) |
| Sodium chloride | 0.15 M |
| Tris(hydroxymethyl)aminomethane | 50 mM |

Here, the phospholipid was made to contain the in the concentration of 7.5 µM, with the phospholipid was prepared by taking respectively into a test tube 0.6 mg phosphatidylserine (pig-brain phosphatidylserine; Ps; Doosan Serdary Research Laboratories Co., South Korea), 0.4 mg phosphatidylcholine (pig-liver phosphatidylcholine; Pc; Doosan Serdary Research Laboratories Co., South Korea), and 1.0 mg phosphatidylethanolamine (pig-liver phosphatidylethanolamine; PE; Doosan Serdary Research Laboratories Co., South Korea), then evaporating chloroform, the solvent, with an evaporator, thereafter adding distilled water and vigorously stirring for 1 min., and then performing sonication at 60°C for 10 min. and contains phosphatidylserine, phosphatidylcholine and phosphatidylethanolamine at the composition ratio of 3 : 2 : 5 (i.e., 30% (W/V) : 20% (W/V) : 50% (W/V)). [Here, the phospholipid with the composition ratio of phosphatidylserine, phosphatidylcholine and phosphatidylethanolamine is 3 : 2 : 5, may be referred to as "phospholipid (PS : PC : PE = 3 : 2 : 5)" below.]

### 2. Specimen

The following (1) to (4) were respectively used as specimens.
(1) Plasma from 211 healthy individuals
(2) Plasma from 7 individuals, who are known to have PS-K155E heterozygote (protein S Tokushima, one of protein S abnormalities), which is gene mutation of protein S
(3) Plasma from one individual, who is known to have PS-K155E homozygote (protein S Tokushima, one of protein S abnormalities), which is gene mutation of protein S
(4) Plasma from one individual, who is known to have C206F heterozygote (one of protein S abnormalities), which is gene mutation of protein S

### 3. Activity Assay for Total Protein S in a Specimen

Activity assay for total protein S in each specimen was conducted using general purpose automated analyzer Model 7170S by Hitachi High-Technologies Co. (Japan).
(1) The specimens in the above-described 2 were respectively diluted with the diluent of the above-described 1(1) at dilution factor of 15 times.
(2) Into 2.0 µL of each specimen diluted with the above-described (1), added was 98 µL of the first reagent of the above-described 1(2), and then the mixture was reacted at 37°C for 1.4 min.
(3) Then, adding 20 µL of the second reagent of the above-described 1(3), the mixture was reacted at 37°C for 8.3 min.
(4) Thereafter, adding 236 µL of the third reagent of the above-described 1 (4), the mixture was reacted at 37°C.
(5) After adding the third reagent of the above-described (4), changes in absorbance at a dominant wavelength of 405 nm and a complimentary wavelength of 505 nm was measured for 12.3 min.

### 4. Calculation of Total Protein S Activity in a Specimen

Differentiating the value of absorbance change (reaction time course) measured in the above-described 3(5) after adding the third reagent, an activity of total protein S in a specimen was determined.

Here, conducting the assay as described in the above 3 for specimens with known total protein S activity, determining a linear equation (differential line) of an absorbance change (reaction time course) per 1 min. of the measured absorbance change after adding the third reagent to time, and plotting the slope of the line against the known total protein S activity, a calibration curve was prepared.

In addition, determining a linear equation (differential line) of the absorbance change per 1min. of absorbance change (reaction time course) after adding the third reagent, which was obtained by conducting the assay on the specimen of the above-described 2 as described in the above 3 to time, and fitting the slope of the line to the above-described calibration curve, an activity value of total protein S in a specimen was determined.

More specifically, determining acceleration of absorbance change (second derivative value of the absorbance) from the absorbance obtained from the measurement, and fitting to the calibration curve, an activity value of total protein S in a specimen was calculated.

The results obtained by conducting the activity assay for total protein S in the specimen are shown in Fig. 1.

II. Assay by a Method and a Reagent for Protein Content of Total Protein S in a Specimen According to Latex Nephelometry

### 1. Activity assay reagent for protein content of total protein S by latex nephelometry

### (1) First reagent

Based on a method by B. Dahlbeack, et al. [Biochem. J., Vol. 209, pp. 847 to 856, 1983], C4b-binding protein was prepared from human plasma.

Then, 50 mM MES-hydrochloric acid buffer solution [pH 6.2 (20°C)], which contains 0.1% (W/V) BSA, 0.3 mol/L sodium chloride, and 0.05% sodium azide, was prepared.

Into the solution, the C4b-binding protein prepared as described above was added to have the concentration of 25 µg/mL, and thereby a first reagent was obtained.

### (2) Second Reagent

### (a) Preparation of Anti-Protein S Antibody

The present inventors prepared anti-protein S antibody according to a normal method, using protein S that is purified and in the free state as immuno antigen.

Performing screening of hybridoma, which produces monoclonal antibody that specifically binds to a site other than a binding site of protein S to the C4b-binding protein, hybridoma of mouse/mouse (9H6 strain) was obtained.

Five mg of mouse anti-protein S monoclonal antibody produced from the hybridoma (9H6 strain) was dissolved in 0.5 mL of sodium phosphate buffer solution (pH 7.5).

In to the mixture, added was 0.01 mL of N,N-dimethyl formamide, in which 0.6 mg of s-acetylmercapto succinic anhydride was dissolved, and the mixture was incubated at room temperature for 30 min.

Subsequently, into the mixture, added were 0.02 mL of 0.1 M EDTA aqueous solution, 0.1 mL of 1 M
tris(hydroxymethyl)aminomethane buffer solution (pH 7.0), and 0.1 mL of 1 M hydroxyl amine hydrochloric acid buffer solution (pH 7.0) respectively, and the mixture was incubated at 30°C for 30 min.

Thereafter, the mixture was subjected to gel filtration with a Sephadex G-25 column, which was equilibrated with 0.1 M sodium phosphate buffer solution (pH 6.0) containing 5 mM EDTA, mercaptosuccinylated mouse anti-protein S monoclonal antibody was obtained.

### (b) Preparation of Anti-Protein S Antibody-Fixed Latex Particles Suspension

Mixing 1.0 mL of 10% suspension of latex particles having average particle diameter of 0.192 µm and 1.0 mL of 50 mM MEC-hydrochloric acid buffer solution [pH 6.0 (20°C)], 32 µL of 320 mM carbodimide (Dojindo Laboratories; Product Number: 348-03631) aqueous solution was further added and mixed, and was left stood on ice for 10 min.

Into 1.2 mL of the above-described latex particle suspension that was left stood on ice for 10 min., added was a 1.8 mL of a solution, in which the mercaptosuccinylated mouse anti-protein S monoclonal antibody prepared in the above-described (a) was mixed in 50 mM MES-hydrochloric acid buffer solution [pH 6.0 (20°C)] at the concentration of 0.083 g/dL, and the mixture was stirred at 4°C overnight.

Then, after removing the supernatant by centrifugation, the precipitated part was suspended in 50 mM Tris-hydrochloric acid buffer solution containing 0.8% BSA [pH 8.0 (20°C)], and was left stood at 37°C for 3 hours to perform blocking treatment.

Subsequently, the precipitated part was collected by centrifugation, then re-dispersed in 0.05% sodium azide aqueous solution containing 0.1% BSA, and suspended so as to have the absorbance of 15, 0 OD at a wavelength of 700 nm.

The resultant was anti-protein S antibody-fixed latex particle suspension.

### (c) Preparation of Second Reagent

The anti-protein S antibody-fixed latex particle suspension prepared in the above-described (b) was diluted to 10 times with 0.05% sodium azide aqueous solution containing 0.1% BSA, and a suspension containing 0.1% "anti-protein S antibody-fixed latex particles" was prepared.

The resultant was a second reagent.

### 2. Specimen

Each of the specimens in the above-described I 2(1) to (4) was used as a specimen.

### 3. Measurement and Calculation of Protein Content of Total protein S in a Specimen

### (1) Measurement Procedure

(a) The measurement was conducted using Toshiba-120FR automated analyzer (manufactured by Toshiba Medical Systems Corporation).
   First, into each test tube (cuvette), added was 3 µL of the specimen in the above-described 2.
   Next, into those test tubes (cuvettes), added and mixed was 100 µL of the above-described 1(1).
   Then, those test tubes (cuvettes) were left stood at 37°C.
   Thereby, a complex of free protein S contained in the above-described specimen and the C4b-binding protein in the above-described first reagent was formed. In other words, protein S contained in the specimen was all turned into "complex of protein S and C4b-binding protein (bound form)".
(b) At the time point of 4 min. 40 sec. after adding the above-described first reagent (at the 16th point), into each mixed solution in those test tubes (cuvettes), 100 µL of the second reagent of the above-described 1(2)(c) was added and mixed.
(c) At the time point of 5 min. 35 sec. after adding the above-described first reagent (at the 19th point), absorbance (wavelength: 700 nm) of each mixed solution in those test tubes (cuvettes) was measured as specimen of a blank test.
   Then, those test tubes (cuvettes) were left stood at 37°C so as to have reaction progressed.
   As such, the antigen-antibody reaction was performed between the anti-protein S antibody fixed on the above-described latex particles and protein S contained the above-described specimen progress, and aggregate of latex particles was formed.
(d) At the time point of 9 min. 47 sec. after adding the above-described first reagent (at the 33rd point), the absorbance (wavelength: 700 nm) of mixed reaction solution in the test tube (cuvette) was measured as a measurement value of the above-described specimen.
(e) Subtracting the absorbance measured in the above-described (c) (specimen blank test) from the absorbance (measurement value) measured in the above-described (d), an absorbance difference was obtained.

Here, the absorbance difference is proportional to the protein content (concentration) of total protein S contained in a specimen.

Making a calibration curve from the absorbance difference of those specimens, and fitting an absorbance difference obtained by measuring an actual specimen (3.2% citrated plasma) by a similar method onto the calibration curve, the protein concentration of total protein S in the specimen was determined.

As such, the protein content of total protein S in each specimen of the above-described 2 was obtained.

The results obtained by performing the assay of protein content of total protein S in the specimen are shown in Fig. 1.

### III. Assay Results

### 1. Description of Drawings

A diagram to compare the assay results of activity values of total protein S in specimens in the above-described I with the protein content of total protein S in the specimens in the above-described II was shown as Fig. 1.

In the figure, horizontal coordinate (x) shows the assay results of protein content of total protein S in the specimens by the latex nephelometry of the above-described II. The unit of the measured value is "µg/mL".

In addition, in the figure, the vertical coordinate (y) shows the assay results of activity values of total protein S in the specimens in the above-described I. The unit of the measured value is "µg/mL equivalent".

### 2. Comparison of Specimens of Healthy Individuals

The comparison between the assay results of activity values of total protein S in the above-described I and the assay results of protein content of total protein S by the latex nephelometry of the above-described II was performed as follows.

For a case that specimens were plasma of 211 healthy individuals, from the measurement results of activity values of total protein s in the above-described I and the measurement results of protein content of total protein S in the above-described II, specific activity (total protein S activity value/total protein S protein content) of protein S was determined.

Calculating a mean value and standard deviation (SD) of a specific activity of protein S in a plasma specimen from 211 healthy individuals, the range of mean ± 2SD (0.98 ± 0.26) was determined.

Since there were 10 specimens that deviated from the range, for the specimens of 201 normal and healthy individuals excluding the ten specimens, the mean value and the standard deviation of the specific activity (total protein S activity value/total protein S protein content) of protein S, which was determined from the assay results of activity values of total protein S and the assay results of protein content of total protein S, were calculated again, and the range of mean ± 2SD (0.99 ± 0.20) and the range of mean ± 3SD (0.99 ± 0.30) were determined.

Here, the figure also shows the specific activity for each specimen, which is obtained by dividing the activity value of total protein S in a specimen by the protein content of total protein S.

In the figure, a line of the equation y = 0.99x, which is a mean value of specific activity is shown with a solid line.

In addition, above and under the line of the equation y = 0.99x, lines indicating mean ± 2SD (y = 1.19x and y = 0.79x) are shown in the figure with broken lines.

Moreover, above the line of the equation y = 0.99x, a line indicating mean + 3SD (y = 1.29x) is shown with a dotted line, and a line indicating mean - 3SD (y = 0.69x) is shown with a solid line below the line of the equation y = 0.99x in the figure.

In the measurement of the plasma specimens of 211 healthy individuals, there were seven that deviated from the above-described line y = 0.69x (mean - 3SD) in the Fig. 1.

In other words, there were seven specimens recognized to have specific activity of not greater than 0.69, with the specific activity was obtained by dividing the activity value of total protein S with the protein content of total protein S.

Among those seven specimens having the specific activity of not greater than 0.69, i.e., among seven specimens in which there is significant gap between the activity value of total protein S and the protein content of total protein S, base sequence of protein S gene was studied for those three specimens that had consents, and it was revealed that any of those three specimens had PS-K155E mutation (one of protein S abnormalities). [In Fig. 1, those three specimens are affixed with an arrow marks (↓ or ↑).]

Accordingly, it was confirmed that it is possible to detect protein S abnormalities such as mutation of protein S gene, by measuring the activity value of total protein S in a specimen and the protein content of total protein S in a specimen, and comparing between the activity value of total protein S and the protein content of total protein S, which were obtained from the measurement, by determining a specific activity, or the like.

### 3. Comparison of Specimens Known to Have Protein S Gene Mutation (Protein S Abnormalities)

For each of specimens (specimens of total 9 individuals) that were known to have gene mutation of protein S (protein S abnormalities) in the above-described I2(2) to (4), comparison was made between the assay result of an activity value of total protein S in the above-described I and the assay result of protein content of total protein S by latex nephelometry of the above-described II.

In Fig. 1, seven plasma specimens, which were known to have PS-K155E heterozygote (one of protein S abnormalities), genetic mutation of protein S in the above-described I2(2), were indicated with "●".

In addition, one plasma specimen, which is known to have PS-K155E homozygote (one of protein S abnormalities), a gene mutation of protein S in the above-described I2(3) is indicated with "◆".

In addition, a plasma specimen of one individual, who was known to have C206F heterozygote (one of protein S abnormalities), gene mutation of protein S in the above-described I2(4), was indicated with "□".

It can be understood that any of those 9 specimens (●, ◆, and □) that were known to have gene mutation of protein S deviated from and was below the line of y = 0.69x (average - 3sD) in the above-described Fig. 1.

More specifically, it can be understood that, in any of those nine specimens (●, ◆, and □) that were known to have gene mutation of protein S, the specific activity that was obtained by dividing the activity value of total protein S by protein content of total protein S was not greater than 0.69.

In other words, it can be understood that, in those 9 specimens, there was significant gap between the activity values of total protein S and the protein content of total protein S.

Therefore, even from the comparison results of the nine specimens having protein S gene mutation, it was confirmed that it is possible detect protein S abnormalities such as mutation of protein S gene by measuring an activity value of total protein S in a specimen and protein content of total protein S in a specimen and comparing the activity value of total Protein S obtained by the measurement and protein content of total protein S by means of determining specific activity or the like.

### [Industrial Applicability]

According to the method of detecting protein S abnormalities of the invention, it is possible to accurately, conveniently, and rapidly detect protein S abnormalities.

Therefore, using the protein S abnormalities detecting method of the invention, it is possible to detect protein S abnormalities conveniently and accurately, and it can serve for prevention diagnosis and treatment of diseases such as thrombosis.

## Claims

1. A method of detecting protein S abnormalities, comprising the steps of:
measuring a total protein S activity and a total protein S protein content in a specimen; and
comparing the total protein S protein activity with the total protein S protein content obtained from the measurement,
wherein the measuring of a total protein S activity comprises the steps of:
(a) contacting said specimen with activated protein C; a surfactant; a phospholipid comprising phosphatidylcholine, phosphatidylserine and phosphatidylethanolamine; calcium ions; activated blood coagulation factor V; activated blood coagulation factor X; prothrombin; and a substrate of thrombin;
(b) measuring the amount of signals produced from the substrate of thrombin as a result of reactions of the respective components of step (a); and
(c) obtaining the activity of total protein S contained in said specimen based on the amount of signals measured in step (b), wherein the production of signals is suppressed according to the activity of total protein S contained in the specimen.

2. The method of detecting protein S abnormalities according to claim 1, further comprising the step of:
determining or suspecting the protein S abnormalities when the total protein S protein activity and the total protein S protein content obtained from the measurement are compared and there is a gap between the total protein S activity and the total protein S protein content.

3. The method of detecting protein S abnormalities according to claim 1 or 2, wherein, when the total protein S protein activity and the total protein S protein content, which are obtained from the measurement obtained from the measurement, are compared, a specific activity is obtained through dividing the total protein S activity by the total protein S protein content.

## Patentansprüche

1. Verfahren zum Bestimmen von Protein-S-Anomalien, aufweisend die Schritte:
Messen einer Gesamt-Protein-S-Aktivität und eines Gesamt-Protein-S-Proteingehalts in einer Probe; und Vergleichen der aus der Messung erhaltenen Gesamt-Protein-S-Proteinaktivität mit dem Gesamt-Protein-S-Proteingehalt,
wobei das Messen einer Gesamt-Protein-S-Aktivität die Schritte aufweist:
(a) in Kontakt bringen der Probe mit aktiviertem Protein-C; einem oberflächenaktiven Stoff; einem Phospholipid aufweisenden Phosphatidylcholin, Phosphatidylserin und Phosphatidylethanolamin; Kalziumionen; aktiviertem Blutgerinnungsfaktor V; aktiviertem Blutgerinnungsfaktor X; Prothrombin; und einem Substrat aus Thrombin;
(b) Messen der Anzahl von Signalen, welche vom Substrat aus Thrombin auf Grund von Reaktionen der entsprechenden Komponenten aus Schritt (a) erzeugt werden; und
(c) Erhalten der Aktivität von in der Probe enthaltenen Gesamt-Protein-S, basierend auf der Anzahl von in Schritt (b) gemessenen Signalen, wobei die Produktion von Signalen in Abhängigkeit von der Aktivität von in der Probe enthaltenem Gesamt-Protein-S unterdrückt wird.

2. Verfahren zum Bestimmen von Protein-S-Anomalien nach Anspruch 1, weiter aufweisend den Schritt:
Feststellen oder Mutmaßen der Protein-S-Anomalien, wenn die von der Messung erhaltenen Gesamt-Protein-S-Proteinaktivität und Gesamt-Protein-S-Proteingehalt verglichen werden und es eine Lücke zwischen der Gesamt-Protein-S-Aktivität und dem Gesamt-Protein-S-Proteingehalt gibt.

3. Verfahren zum Bestimmen von Protein-S-Anomalien nach Anspruch 1 oder 2, wobei, wenn die Gesamt-Protein-S-Proteinaktivität und der Gesamt-Protein-S-Proteingehalt, die von der Messung erhalten werden, verglichen werden, eine spezifische Aktivität erhalten wird, indem die Gesamt-Protein-S-Aktivität durch den Gesamt-Protein-S-Proteingehalt dividiert wird.

## Revendications

1. Une méthode de détection d'anomalies de la protéine S comprenant les étapes consistant à :
mesurer l'activité totale de la protéine S et la quantité totale de protéine S dans un échantillon ; et comparer l'activité totale de la protéine S à la quantité totale de protéine S obtenues grâce à la mesure,
la mesure de l'activité totale de la protéine S comprenant les étapes consistant à :
(a) mettre en contact ledit échantillon avec une protéine C activée ; un tensioactif ; un phospholipide comprenant de la phosphatidylcholine, de la phosphatidylsérine et de la phosphatidyléthanolamine ; des ions de calcium ; le facteur V activé de coagulation sanguine ; le facteur X activé de coagulation sanguine ; de la prothrombine ; et un substrat de thrombine ;
(b) mesurer la quantité de signaux produits par le substrat de thrombine résultant des réactions des composants respectifs énoncés à l'étape (a) ; et
(c) obtenir l'activité totale de la protéine S dudit échantillon en se fondant sur la quantité de signaux mesurés à l'étape (b), la production de signaux étant inhibée en fonction de l'activité de la protéine S totale contenue dans l'échantillon.

2. La méthode de détection d'anomalies de la protéine S selon la Revendication 1 comprenant en outre les étapes consistant à :
déterminer ou suspecter les anomalies de protéine S lorsque l'activité de protéine de la protéine S totale et la quantité de protéine de la protéine S totale obtenues grâce aux mesures sont comparées et qu'il y a un écart entre l' activité de protéine de la protéine S totale et la quantité de protéine de la protéine S totale.

3. La méthode de détection d'anomalies de la protéine S selon la Revendication 1 ou 2 dans laquelle,
lorsque l'activité de protéine de la protéine S totale et la quantité de protéine de la protéine S totale obtenues grâce aux mesures sont comparées, une activité spécifique est obtenue en divisant l'activité de protéine de la protéine S totale par la quantité de protéine de la protéine S totale.
